# DEMANDE DE BREVET EUROPEEN

(11) **EP 1 151 996 A2**
(43) Date de publication de la demande: **07.11.2001**
(21) Numéro de dépôt: 01401107.6
(22) Date de dépôt: 27.04.2001
(51) Int. Cl.: C07D 227/087

(54) **Procédé de préparation des N,N'-carbonyl bis lactames**

(30) Priorité: 28.04.2000 FR 0005502
(71) Demandeur: SNPE, 75181 Paris Cedex 04 (FR)
(72) Inventeur: Bonnard, Hubert, 91590 Cerny (FR); Ferruccio, Laurence, 91810 Vert Le Grand (FR); Leroy, Pierre-Yves, 31120 Roques sur Garonne (FR); Senet, Jean-Pierre, 77760 Buthiers (FR)

(57) **Abrégé**

L'invention concerne un procédé de préparation des N,N'-carbonylbislactames par réaction du phosgène avec au moins un lactame qui est caractérisé en ce que l'amine tertiaire est choisie dans le groupe constitué par les amines tertiaires aliphatiques non nucléophiles.

On obtient ainsi des N,N'-carbonylbislactames de pureté élevée et avec de bons rendements aussi bien au laboratoire qu'à l'échelon industriel.

## Description

L'invention concerne la préparation des N,N'-carbonylbislactames. Elle concerne en particulier un procédé amélioré de préparation de ceux-ci à partir de lactames et de phosgène.

Les procédés de préparation des N,N'-carbonylbis lactames sont peu nombreux. Dans la majorité d'entre eux, le phosgène est utilisé. Le deuxième réactif est alors un lactame ou un de ses dérivés tel qu'un sel alcalin ou un dérivé triméthylsilylé. Aucun de ces procédés ne donne satisfaction.

Ainsi lorsqu'on fait réagir le phosgène avec le sel de sodium de l'∈-caprolactame selon H. R. Meyer comme décrit dans le résumé CA 52 : 11781e de 1956, on obtient de 0,6 à 40% de N,N'-carbonylbiscaprolactame seulement.

Selon une autre méthode telle que décrite dans l'article CA 68 :104571, l'∈-caprolactame et le phosgène sont mis à réagir en présence d'une amine non protonique telle que la triéthylamine, à une température de 20°C puis de 40°C. Le rendement à l'échelon laboratoire serait un peu plus élevé, d'environ 60%. Cependant lorsqu'on reproduit cet exemple, le rendement obtenu n'est que de 40%. De plus, si l'on utilise des quantités plus importantes de réactifs par exemple convenant pour un réacteur de 50 litres, le rendement n'est alors plus que de 23%.

Dans le brevet US 5 972 237, la même méthode de préparation du N,N'-carbonylbiscaprolactame est employée. L'amine utilisée est la diméthylcyclohexylamine. Le rendement indiqué n'est alors que de 46%.

Selon un autre procédé (Polymer Journal, 1995, 27(5), pp 449-450), on forme tout d'abord le dérivé N-triméthylsilylé de l'∈-caprolactame puis on fait réagir une partie de celui-ci avec le phosgène pour former le chlorure de carbamoyle. Les deux composés intermédiaires sont ensuite mis à réagir l'un avec l'autre pour obtenir le carbonylbiscaprolactame. Trois étapes sont par conséquent nécessaires. Le rendement n'est pas mentionné. Lorsqu'on reproduit ce procédé, on trouve un rendement de 51%.

Les N,N'-carbonylbislactames sont des composés très utiles en particulier comme co-catalyseurs et activateurs dans la fabrication de polymères tels que les polyamides ou polyesters, comme activateurs de peroxydes minéraux dans les lessives ou comme intermédiaires dans la synthèse d'aminoacides. Ils doivent pour la plupart de leurs applications être d'une grande pureté.

Il existait par conséquent un besoin d'obtenir les N,N'-carbonylbislactames avec un bon rendement, non seulement au laboratoire mais également à l'échelon industriel, et dans des conditions économiques.

Un objet de la présente invention est également de les obtenir avec une grande pureté.

Le procédé selon l'invention résout les problèmes précédemment indiqués. Il consiste à faire réagir le phosgène avec au moins un lactame en présence d'une amine tertiaire choisie dans le groupe constitué par les amines tertiaires aliphatiques non nucléophiles.

Les N,N'-carbonylbislactames sont obtenus grâce à ce procédé généralement avec un rendement supérieur à 70% et une pureté supérieure à 99%.

Le procédé est particulièrement intéressant pour transformer les lactames de formule (I) 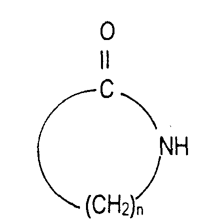 dans laquelle n représente un nombre entier de 3 à 15 et de préférence de 5 à 12.

Les N,N'-carbonylbislactames obtenus ont pour formule (II)

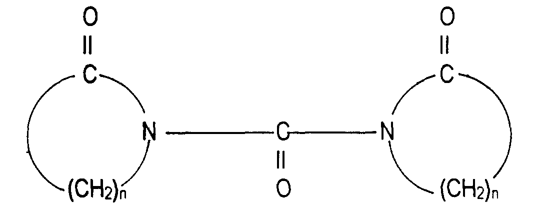

Lorsqu'un seul lactame est utilisé, les deux cycles du composé sont identiques. Lorsqu'on utilise comme composés de départ plusieurs lactames différents, on obtient un mélange de N,N'-carbonylbislactames avec des cycles identiques ou différents.

Le phosgène est mis à réagir avec le ou les lactames, en général, en quantité stoechiométrique ou voisine de cette valeur, soit aux environs de 0,5 mole de phosgène par mole de lactame. Un grand excès molaire de phosgène n'est pas nécessaire. De préférence; on utilise de 0,5 à 0,55 mole de phosgène par mole de lactames.

De façon particulièrement inattendue, on a trouvé que la nature de l'amine était très importante pour résoudre les problèmes qui se posaient pour l'obtention des N,N'-carbonylbislactames, dans de bonnes conditions.

L'amine doit être sélectionnée parmi les amines tertiaires aliphatiques qui ne sont pas ou quasiment pas nucléophiles, c'est à dire qui ne sont pas ou quasiment pas réactives vis à vis d'espèces électrophiles comme par exemple le groupe carbonyle. Ces amines sont généralement des amines possédant des radicaux très encombrants qui empêchent ainsi le doublet d'électrons libres de l'azote d'être accessible.

Il est préférable que les amines soient également fortement basiques.

Les amines utiles sont en particulier les amines de formule NR¹R²R³ dans laquelle R¹ représente le radical méthyle ou éthyle tandis que R² et R³, identiques ou différents, représentent le radical isopropyle ou isobutyle. Comme exemple de telles amines, on peut citer la diisopropylméthylamine, la diisopropyléthylamine, la diisobutyléthylamine. De préférence, on utilise la diisopropyléthylamine.

La présence d'une quantité d'amine suffisante pour piéger l'acide chlorhydrique qui se forme au cours de la réaction est nécessaire. En général, on utilise aux environs de 1 mole d'amine par mole de lactame et de préférence de 0,95 à 1,05 mole par mole de lactame. Un excès d'amine n'est pas utile.

Selon les procédés précités de l'art antérieur dans lesquels le phosgène est utilisé, celui-ci est introduit dans le milieu réactionnel, à une température d'environ 20°C. On a maintenant trouvé que l'on améliorait encore le procédé selon l'invention en introduisant le phosgène dans le milieu réactionnel à une température comprise entre environ - 10°C et + 5°C et de préférence comprise entre environ - 5°C et 0°C. Les rendements sont meilleurs et la pureté encore plus élevée. L'introduction du phosgène dure en général une à plusieurs heures.

La réaction est généralement réalisée dans un solvant organique inerte vis à vis des réactifs et dont le point de fusion est inférieur à la température d'introduction du phosgène, en particulier inférieur à environ - 10°C. De préférence, le point d'ébullition du solvant est inférieur à environ 160°C. Les solvants aromatiques tels que le toluène et le xylène conviennent bien.

Lorsque toute la quantité de phosgène a été introduite, on laisse se poursuivre la réaction à une température d'environ 40° à 50°C, généralement pendant quelques heures.

Pour récupérer les N,N'-carbonylbislactames obtenus, on sépare en général du milieu le chlorhydrate de l'amine qui s'est formé, par exemple par filtration ou par lavage à l'eau suivi de la récupération de la phase aqueuse par décantation, puis on élimine le solvant, notamment par évaporation sous pression réduite. On peut ajouter éventuellement ensuite dans le milieu un composé non solvant des carbonylbislactames, tel que l'isopropanol, le méthanol ou l'eau, puis on les fait précipiter. Si nécessaire, on peut de plus les purifier par lavage avec un alcool, par exemple tel que le méthanol, et/ou effectuer une recristallisation.

Les rendements obtenus grâce au procédé de l'invention sont nettement améliorés par rapport à ceux de l'art antérieur. Ainsi le N,N'-carbonylbiscaprolactame peut être obtenu avec un rendement au laboratoire supérieur à 80% et à l'échelle industrielle supérieur à 70%. La pureté est excellente et en général supérieure à 99%.

Le procédé selon l'invention est particulièrement économique car on a aussi trouvé un moyen de récupérer la diisopropyléthylamine avec une grande pureté à partir de son chlorhydrate formé au cours de la réaction. Pour ce faire, on forme une solution aqueuse du chlorhydrate de l'amine avec de l'eau, de préférence très pure, soit après avoir séparé le chlorhydrate du milieu réactionnel, soit en ajoutant l'eau dans le milieu et en récupérant la phase aqueuse, par exemple par décantation, puis on neutralise cette solution avec une base minérale alcaline telle que la lithine, la soude ou la potasse, en particulier en solution aqueuse. La soude est la base préférée.

On utilise en général une quantité de base telle que le pH final de la solution se situe aux environs de 13.

On effectue alors une codistillation de la solution, généralement à une température comprise entre environ 83° et environ 91°C à la pression normale. Du mélange récupéré, l'amine se sépare par décantation. Elle a généralement une pureté, déterminée par analyse CPG, égale ou supérieure à 99% et peut être utilisée dans un nouveau cycle de production de N,N'-carbonylbislactames.

Les exemples qui suivent illustrent l'invention sans toutefois la limiter.

### EXEMPLE 1 : Préparation du N,N'-carbonylbis(∈-caprolactame) (dénommé CBC).

Dans un réacteur double enveloppe équipé d'un agitateur mécanique tournant à 300 tr/min, on introduit 2,1 kg de toluène, 277 g (2,426 mol) d'∈-caprolactame et 318 g (2,43 mol) de diisopropyléthylamine (dénommé DIEA). On refroidit le mélange à - 5°C et on introduit régulièrement 129 g (1,3 mol) de phosgène gazeux au sein du mélange, à un débit d'environ 75 g par heure, en maintenant la température entre - 5° et 0°C.

L'introduction du phosgène terminée, on chauffe le milieu réactionnel à 40°-43°C, et on le maintient à cette température sous agitation pendant 5 h.

On le refroidit ensuite à environ +10°C puis on sépare le précipité formé par filtration. On rince le gâteau avec 499 g puis 402 g et 491 g de toluène. On récupère alors 447,9g de chlorhydrate de DIEA humide.

Les filtrats obtenus sont rassemblés et constituent une phase toluénique unique de 3,629 kg. On concentre alors le mélange en distillant 3,029 kg de toluène à 35°C sous 14-25 mm de Hg. Le CBC précipite en cours de distillation mais le milieu reste agitable.

On ajoute alors 1,8 kg d'isopropanol dans le milieu et on distille 1,875 kg d'un mélange isopropanol/ toluène (84/16 en masse) à 62°-43°C sous 381 à 133 mm de Hg.

On chauffe ensuite le milieu à 76°C, le CBC se redissout. On refroidit la solution à 0°-5°C, le CBC précipite vers 45°C.

On filtre le mélange, on lave le gâteau avec 53 g puis 47 g d'isopropanol à 0°-5°C. On sèche ensuite les gros cristaux blancs obtenus en étuve sous pression réduite (10 mm Hg, 70°C) pendant 1 heure.

On obtient ainsi 248 g de N,N'-carbonylbis(∈-caprolactame) (rendement 81% par rapport à l'∈-caprolactame de départ) de pureté 100% (dosage HPLC) et dont la teneur en chlorure est indétectable démontrant ainsi l'absence de produits gênants tels que le chlorure de carbamoyle ou des sels organiques de type Vilsmeier-Haack.

### EXEMPLE 2 : Préparation du N,N'-carbonylbis(∈-caprolactame).

On opère comme à l'exemple 1, mais en utilisant 300 ml de toluène, 0,402 mol d'∈-caprolactame, 0,404 mol de DIEA et 0,21 mol de phosgène et en introduisant le phosgène en 30 min, tout en maintenant la température du milieu réactionnel entre 10° et 17°C.

On obtient le N,N'-carbonylbis(∈-caprolactame) avec un rendement de 77% et une pureté de 98% (dosage HPLC).

### EXEMPLE COMPARATIF : Préparation du N,N'-carbonylbis(∈-caprolactame) par phosgénation en présence de triéthylamine.

On opère comme à l'exemple 2, mais en remplaçant la DIEA par 0,42 mol de triéthylamine.

On obtient alors le N,N'-carbonylbis(∈-caprolactame) avec un rendement de 14% seulement et une pureté de 95% (dosage HPLC).

### EXEMPLE 3 : Préparation du N,N'-carbonylbis(∈-caprolactame).

On opère comme à l'exemple 1, mais en utilisant 400 ml de toluène, 0,403 mol de ∈-caprolactame, 0,404 mol de DIEA et 0,25 mol de phosgène.

On obtient le N,N'-carbonylbis(∈-caprolactame) avec un rendement de 70% et une pureté de 98% (dosage HPLC).

### EXEMPLE 4 : Récupération et recyclage de la diisopropyléthylamine utilisée à l'exemple 1.

Dans un réacteur double enveloppe de 0,5 1, équipé d'un Dean Stark à recyclage de phase inférieure et d'un condenseur à serpentin alimenté par du glycol à - 15°C, on introduit 95 g d'eau distillée et 145,7 g (0,87 mol) de chlorhydrate de DIEA obtenu à l'exemple 1. La dissolution du chlorhydrate est totale en 15 min à 20°C.

On ajoute alors 114 g (0,914 mol) d'une solution aqueuse de soude à 32%. La neutralisation est endothermique. On ajuste le pH final de la solution à 13 ± 0,5.

On codistille à la température de 83°-91°C (température de masse 103°-109°C) sous pression normale. On obtient alors un mélange constitué d'une phase aqueuse (87 g) et de DIEA qui se sépare par décantation. On récupère ainsi 111,3 g de DIEA de pureté 99% (analyse CPG), contenant 0,14% d'eau et ne contenant pas de diisopropylamine. Le rendement est de 98% par rapport au chlorhydrate de DIEA de départ.

On utilise cette DIEA pour préparer du CBC selon le mode opératoire de l'exemple 1. On obtient alors le N,N'-carbonylbis(∈-caprolactame) avec un rendement de 83% et une pureté supérieure à 99,7% (dosage HPLC).

### Exemple 5 : Préparation du N,N'-carbonylbis(∈-caprolactame).

Dans un réacteur de 20 l agité, on met en suspension 2,2 kg d'∈-caprolactame (19,4 mol) dans 6,6 kg de toluène (soit une concentration en caprolactame de 25 %). 2,59 kg de diisopropyléthylamine (DIEA, 20,0 mol, 1,03 eq) sont ensuite ajoutés à température ambiante. Le milieu est refroidi à 0°C, et 1,0 kg de phosgène gazeux (10,1 mol, 0,52 eq) sont alors introduits dans le milieu, la température du réacteur restant inférieure à 12°C. L'introduction terminée, le milieu est chauffé à 40°-50°c pendant deux heures puis 4,4 kg d'eau sont alors ajoutés dans le milieu en 30 minutes entre 50° et 40°C. Le chlorhydrate de DIEA se solubilise immédiatement. Après 30 minutes d'agitation, celle-ci est stoppée, et les phases homogènes sont décantées pendant 30 minutes. La phase aqueuse (7,8 kg) est soutirée et conservée pour le retraitement du chlorhydrate de DIEA. La phase toluénique est chauffée et mise sous vide pour effectuer la concentration (à 35°-50°C, sous 80-50 mm Hg). En 1,5 heure, 4,8 kg de toluène sont distillés, puis le vide est cassé et 5 kg d'eau sont coulés dans le milieu. Le CBC cristallise. Le réacteur est remis sous vide (à 65°-70°C, sous 450-400 mm Hg) pour distiller l'azéotrope eau/toluène en vue de l'épuisement du milieu en toluène. Après 3,5 h de distillation, 960 g de toluène et 140 g d'eau sont récupérés dans les distillats. 4,8 kg de méthanol sont alors ajoutés dans le milieu à la pression atmosphérique et le milieu est agité pendant 1h à 40°C. Il est ensuite refroidi à 5°C, et la suspension est envoyée sur le filtre. 8,4 kg de jus mères sont récupérés, et le gâteau est lavé avec 2,5 kg d'un mélange 50/50 d'eau et de méthanol (2,55 kg de jus de lavage sont récupérés). 1,9 kg de CBC humide sont isolés sur le filtre. Après 6 h de séchage (à 50°C, sous 10 mm Hg), on obtient 1,85 kg (rendement 76 %) de CBC sec de pureté supérieure à 99,5 % (% p/p, HPLC) et avec un point de fusion de 115°C.

## Revendications

1. Procédé de préparation des N,N'-carbonylbislactames par réaction du phosgène avec au moins un lactame en présence d'une amine tertiaire, **caractérisé en ce que** l'amine tertiaire est choisie dans le groupe constitué par les amines tertiaires aliphatiques non nucléophiles.

2. Procédé selon la revendication 1, **caractérisé en ce que** les lactames de départ sont représentés par la formule générale (I) 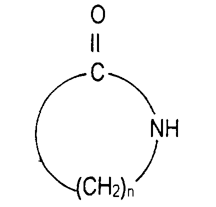 dans laquelle n représente un nombre entier de 3 à 15.

3. Procédé selon la revendication 1 ou 2, **caractérisé en ce que** l'amine est choisie parmi les amines de formule NR¹R²R³ dans laquelle R¹ représente le radical méthyle ou éthyle et R² et R³, identiques ou différents, représentent le radical isopropyle ou isobutyle.

4. Procédé selon l'une quelconque des revendications précédentes, **caractérisé en ce que** l'amine est la diisopropyléthylamine.

5. Procédé selon l'une quelconque des revendications précédentes, **caractérisé en ce que** le phosgène est introduit dans le milieu réactionnel à une température comprise entre environ - 10°C et environ + 5°C.

6. Procédé selon l'une quelconque des revendications précédentes, **caractérisé en ce que**, après l'introduction du phosgène, le mélange réactionnel est chauffé à une température comprise entre environ 40° et environ 50°C.

7. Procédé selon l'une quelconque des revendications précédentes, **caractérisé en ce que** la réaction est effectuée dans un solvant choisi parmi les solvants de point de fusion inférieur à environ - 10°C.

8. Procédé selon l'une quelconque des revendications 4 à 7, **caractérisé en ce que**, la réaction étant terminée, pour récupérer la diisopropyléthylamine, on dissout dans l'eau le chlorhydrate de diisopropyléthylamine formé, en ajoutant l'eau dans le milieu réactionnel ou après avoir séparé le chlorhydrate du milieu, on neutralise la solution aqueuse obtenue, le cas échéant séparée du milieu réactionnel, avec une base minérale alcaline puis on effectue une codistillation du mélange.

9. Procédé selon la revendication précédente, **caractérisé en ce que** la quantité de base ajoutée est telle que le pH final se situe aux environs de 13.

10. Procédé selon la revendication 8 ou 9, **caractérisé en ce que** la température de codistillation est comprise entre environ 83°C et 91°C.

11. Procédé selon l'une quelconque des revendications précédentes, **caractérisé en ce qu'**on récupère le N,N'-carbonylbislactame par cristallisation dans l'eau.

12. Procédé selon l'une quelconque des revendications précédentes, **caractérisé en ce qu'**on purifie le N,N'-carbonylbislactame par lavage avec un alcool.
